(19) Europäisches Patentamt
European Patent Office
Office européen des brevets

(11) Veröffentlichungsnummer: **0 270 841**
**A2**

(12) **EUROPÄISCHE PATENTANMELDUNG**

(21) Anmeldenummer: **87116300.2**

(51) Int. Cl.4: **C07C 121/66**, C11B 9/00

(22) Anmeldetag: **05.11.87**

(30) Priorität: **15.11.86 DE 3639158**

(43) Veröffentlichungstag der Anmeldung:
**15.06.88 Patentblatt 88/24**

(84) Benannte Vertragsstaaten:
**CH DE ES FR GB LI NL**

(71) Anmelder: **BASF Aktiengesellschaft**
**Carl-Bosch-Strasse 38**
**D-6700 Ludwigshafen(DE)**

(72) Erfinder: **Martin, Roland, Dr.**
**10 Im Mosenborn**
**D-6701 Kallstadt(DE)**
Erfinder: **Gramlich, Walter, Dr.**
**11 Auf der Hoehe**
**D-6803 Edingen-Neckarhausen(DE)**

(54) **2-Methyl-3-(p-methyl-phenyl)-propionitril, dessen Herstellung und Verwendung als Riechstoff.**

(57) 2-Methyl-3-(p-methyl-phenyl)propionitril, dessen Herstellung und Verwendung als Riechstoff.

EP 0 270 841 A2

BASF Aktiengesellschaft O.Z. 0050/38785

2-Methyl-3-(p-methyl-phenyl)-propionitril, dessen Herstellung und Verwendung als Riechstoff

Beschreibung

Auf dem Gebiet der Parfümerie und der Aromaentwicklung besteht trotz der großen Zahl der bereits bekannten natürlichen und synthetischen Riechstoffe nach wie vor ein großer Bedarf an neuen Riechstoffen, die entweder bisher unbekannte Geruchsnoten aufweisen oder aber teure bzw. schwierig zugängliche Riechstoffe ersetzen können. Ursache ist der ständig wachsende Bedarf an Parfümkompositionen, sowohl für die Feinparfümerie als auch für kosmetische und technische Produkte wie Waschmittel, Weichspüler und dergl.

Verbindungen, die einen reinen Jasmingeruch besitzen, sind stark gefragt, da die natürliche Jasminbase nicht unbegrenzt zur Verfügung steht. Es sind daher sehr viele Versuche unternommen worden, die Jasmininhaltstoffe zu synthetisieren (vgl. E.P. Demole, "The Fragrance of Jasmine" in "Fragrance Chemistry", Herausgeber E.T. Theimes, Academic Press 1982, Seiten 362-396). Diese Naturstoffe sind jedoch nicht auf einfache Weise herzustellen.

Es sind auch schon rein synthetisch hergestellte Verbindungen mit Jasmin-ähnlicher Duftnote bekannt geworden, die in ihrer Struktur nichts mit den natürlichen Jasmininhaltsstoffen gemein haben (vgl. loc. cit. Seiten 387-90). Genannt seien beispielsweise 2-Benzyliden-heptanal, 2-Benzyliden-octanal, 2-Furfuryliden-heptanal, 2-Acetyl-oc-tan-säureethylester, Tetrahydropyran-4-yl-acetate und 1,3-Dioxane mit einer linearen $C_5$- bis $C_7$-Seitenkette. Nachteilig an den bekannten Jasminriechstoffen ist, daß entweder der Jasmingeruch nicht rein ist (2-Acetyl-octan-säureethylester), oder aber die Haftfestigkeit und/oder die chemische Stabilität zu wünschen übrig lassen (Benzylidenoctanal und Benzylidenheptanal).

Es bestand daher die Aufgabe Verbindungen zu synthetisieren, die einfach zugänglich sind, einen langanhaftenden reinen Jasmingeruch besitzen sowie eine große Stabilität in verschiedenen Anwendungsbereichen aufweisen.

Es wurde nun überraschenderweise gefunden, daß das neue 2-Methyl-3-(p-methyl-phenyl)-propionitril der Formel I

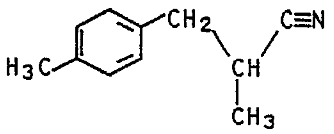

(I)

eine Verbindung mit einem besonders reinen Jasmingeruch darstellt, der darüber hinaus in einem weiten pH-Bereich extrem stabil ist. Ein weiterer besonderer Vorteil des neuen Jasminriechstoffs I ist seine außergewöhnlich hohe Haftfestigkeit (etwa 7 Tage nach Versuchen auf Riechstreifen).

Gegenstand der Erfindung ist dementsprechend neben der neuen Verbindung I seine Verwendung als Riechstoff sowie Riechstoffkompositionen enthaltend diese Verbindung I.

I ist eine neue Verbindung. Einige Verbindungen mit ähnlicher Struktur sind zwar schon aus der Literatur bekannt, jedoch wurden sie ausschließlich als Zwischenprodukte für die Synthese anderer Verbindungen erwähnt.

So ist beispielsweise das 2-Methyl-3-(p-tert.-butyl-phenyl)-propionitril (CAS-Nr. 93 981-807) aus Research Disclosure 225 -001 (Januar 1983) als Zwischenprodukt für die Herstellung von 2-Methyl-3-(p-tert.butyl)-propionaldehyd bekannt. Ferner ist 2-Methyl-3-(p-isopropyl-phenyl)-propionitril (CAS-Nr. 67845-51-6) bekannt.

Weiterhin ist das 2-Methyl-3-(p-methoxy-phenyl)-propionitril aus C.A. Vol. 80 (1974) 108 143q und C.A. Vol. 78 (1973) 84009f als Zwischenprodukt für die Herstellung von 2-Methyl-3-(p-methoxy-phenyl)-ethylamin bekannt. Wie eigene Untersuchungen gezeigt haben, besitzt dieses Nitril einen blumigen, fruchtigen Geruch, der aber schwächer ist als der des entsprechenden Aldehyds, der unter dem Handelsnamen Canthoxal® als Riechstoff im Handel ist.

Die Herstellung des erfindungsgemäßen Riechstoffs I erfolgt in an sich bekannter Weise z.B. durch Umsetzen von 2-Methyl-3-(p-methyl-phenyl)-propanal mit Hydroxylamin und Dehydratisieren des erhaltenen Oxims mit Essigsäureanhydrid (vgl. R. DeSimone "Nitriles in Perfumery", in Perfumer & Flavorist Vol. 4 (1980), Seiten 1-8, insbesondere Seite 6). 2-Methyl-3-(p-methyl-phenyl)-propanal ist selbst ein gut verfügbarer Riechstoff mit einem starken Duft nach Orangen und einer leichten Jasmin-Note. Es war überraschend, daß durch die Überführung des Aldehyds in das entsprechende Nitril der dominierende Orangenduft total verschwindet und ein starker und sehr reiner Jasmingeruch erhalten wird.

Das gilt besonders, da in loc. cit. auf Seite 7, linke Spalte ausgeführt wird, daß Nitrile sehr häufig einen analogen Geruch aufweisen wie die entsprechenden Aldehyde oder gar völlig geruchlos sind, und daß man keine rechte Erklärung hat für große Unterschiede im Geruch zwischen Aldehyden und Nitrilen.

Ein besonderer Vorteil der erfindungsgemäß als Riechstoff verwendeten Verbindung I ist neben dem reinen Jasmingeruch die hohe Haftfestigkeit (~ 7 Tage) sowie ihre chemische Stabilität besonders in basischem Milieu (pH 14), so daß die erfindungsgemäße Verbindung nicht nur in der Feinparfümerie eingesetzt, sondern auch breite Anwendung in Seifen, Wasch- und Reinigungsmittel finden kann.

Sie läßt sich sehr gut mit den üblichen Parfüminhaltsstoffen und anderen Riechstoffen zu neuartigen Kompositionen kombinieren, denen sie ebenfalls eine hohe Haftfestigkeit verleiht. Der Anteil an I in den Riechstoffkompositionen beträgt im allgemeinen 1 - 50 Gewichtsprozent. Derartige Kompositionen können zur Parfümierung von kosmetischen Präparaten wie Cremes, Lotionen, Duftwässern, Aerosolen, Toilettseifen, Mundpflegemitteln sowie in der Extrait-Parfümerie verwendet werden; daneben können sie ferner zur Geruchsverbesserung technischer Produkte wie Weichspüler, Wasch- und Reinigungsmitteln verwendet werden.

Beispiel

In einem Kolben wurden 93 g (1,34 m) Hydroxylammoniumchlorid in 170 ml Wasser gelöst. Dazu wurden 267 g einer 20 %igen Natronlauge getropft. Nach beendeter Zugabe wurde die Lösung 10 Minuten (min) gerührt und danach 162 g (1 m) 2-Methyl-3-(p-methyl-phenyl)-propanal zugetropft. Anschließend ließ man das Reaktionsgemisch 12 Stunden (h) bei Raumtemperatur rühren, gab dann 400 ml Toluol zu und rührte weitere 30 min. Die organische Phase wurde abgetrennt, 2 x mit je 150 ml Wasser gewaschen, getrocknet und eingedampft.

Das rohe Oxim wurde während 1 h bei 100°C zu 680 g (6,66 m) Essigsäureanhydrid zugetropft. Danach wurde noch 3 h unter Rückflußtemperatur zum Sieden erhitzt. Anschließend wurde auf 80°C abgekühlt und während 3-4 h mit 330 ml Wasser hydrolysiert. Die Mischung wurde unter Rühren auf Raumtemperatur abgekühlt und 3 x mit je 250 ml Toluol extrahiert. Die organischen Phasen wurden vereinigt und 2 x mit je 150 ml Wasser sowie 100 ml einer 10 %igen Natronlauge neutral gewaschen, eingedampft und destilliert. Man erhielt 126 g entsprechend 79 % der Theorie) an 2-Methyl-3-(p-methyl-phenyl)-propionitril vom

Kp = 77-93°C/0,4 mbar

$d_{20}^{4}$ = 0,9588 g/cm³; $n_D^{20}$ = 1,5100

MS:$M^+$ = 159 (8 %), 105 (100 %), 77 (10 %) m/e

IR (Film): ν(C≡N) 2259, 1516, 1455, 819, 792, 753 $cm^{-1}$

$^1$H-NMR($CDCl_3$): δ = 1,25 (d, 3H), 2,3 (s, 3H), 2,75 (m, 3H), 7,1 (m, 4H, ppm

$^{13}$C-NMR($CDCl_3$): δ = 17,5 (Q), 20,9 (Q), 27,48 (d), 39,47 (t), 122,55 (S), 128,9 (d), 129,8 (d), 133,9 (S), 136,6 (S) ppm

Duftnote: feiner, reiner Jasmingeruch.

Das Jasmingeruch war 7 Tage nach Auftragen auf Riechpapier noch gut wahrnehmbar.

Patentansprüche

1. 2-Methyl-3-(p-methyl-phenyl)-propionitril.

2. Verfahren zur Herstellung von 2-Methyl-3-(p-methyl-phenyl)-propionitril gemäß Anspruch 1, dadurch gekennzeichnet, daß man 2-Methyl-3-(p-methyl-phenyl)-propanal in an sich bekannter Weise mit Hydroxylamin in das entsprechende Oxim umsetzt und dieses mit Acetanhydrid spaltet.

3. Verwendung von 2-Methyl-3-(p-methyl-phenyl)-propionitril als Riechstoff.

4. Riechstoffkompositionen, gekennzeichnet durch einen Gehalt an 2-Methyl-3-(p-methyl-phenyl)-propionitril.

5. Riechstoffkompositionen gemäß Anspruch 4, dadurch gekennzeichnet, daß sie 2-Methyl-3-(p-methyl-phenyl)-propionitril in einer Menge von 1 bis 50 Gewichtsprozent, bezogen auf die gesamte Komposition enthalten.